# EUROPEAN PATENT SPECIFICATION

(11) **EP 3 025 665 B1**
(45) Date of publication and mention of the grant of the patent: **01.11.2017**
(21) Application number: 15196612.4
(22) Date of filing: 26.11.2015
(51) Int. Cl.: A61B 34/20, A61B 90/00

(54) **COMPUTER-ASSISTED SYSTEM FOR GUIDING A SURGICAL/DIAGNOSTIC INSTRUMENT IN THE BODY OF A PATIENT**
COMPUTERGESTÜTZTES SYSTEM ZUM FÜHREN EINES CHIRURGISCHEN/DIAGNOSTISCHEN INSTRUMENTS IM KÖRPER EINES PATIENTEN
SYSTÈME ASSISTÉ PAR ORDINATEUR PERMETTANT DE GUIDER UN INSTRUMENT DE DIAGNOSTIC/CHIRURGICAL DANS LE CORPS D'UN PATIENT

(30) Priority: 26.11.2014 IT TO20140974
(43) Date of publication of application: 01.06.2016
(73) Proprietor: MASMEC S.p.A., 70026 Modugno (IT)
(72) Inventor: LARIZZA, Piero, 70026 MODUGNO (IT)
(74) Representative: Bongiovanni, Simone

(56) References cited:
- WO-A1-2005/076033
- WO-A1-2014/005225
- US-A- 5 999 840
- US-A1- 2002 002 330
- US-A1- 2004 147 839
- US-A1- 2007 280 423
- US-A1- 2013 060 146
- NDI: "The Polaris Family of Products", INTERNET CITATION, July 2008 (2008-07), pages 1-8, XP002619770, Retrieved from the Internet: URL:http://www.ndigital.com/medical/docume nts/polaris/polarisspectra/Comparison_Pola ris_and_Polaris_Spectra.pdf [retrieved on 2011-01-31]

## Description

### TECHNICAL FIELD OF THE INVENTION

The present invention relates to a computer-assisted system for guiding a surgical/diagnostic instrument in the body of a patient.

### BACKGROUND ART

As is known, many surgical and/or diagnostic operations are conducted with the aid of a surgical/diagnostic instrument (operating tool) able to reach, percutaneously, an area affected by a pathology (often indicated as target area). By way of example, these operations could be biopsies, thermal ablation, removal of damaged tissue, the introduction of chemical/pharmaceutical products into the human body. Introduction and subsequent reaching of the target by the surgical/diagnostic instrument can be facilitated by guide, or navigation, systems, of virtual type, based on images of the target and of the areas surrounding the target so as to plan and perform the percutaneous operation in the least invasive way.

Known navigation/guide systems for operations of the aforesaid type are based on the processing of images acquired previously through computerized tomography CT or magnetic resonance MR and require the definition of a virtual reality three-dimensional reconstruction of the target area of the human body.

A three-dimensional representation of the surgical/diagnostic instrument is superimposed on the three-dimensional reconstruction and moved on the image following the real movements of the instrument.

It is evident that in order to view the position in space of the surgical/diagnostic instrument this must be provided with suitable sensors so that the position of the surgical/diagnostic instrument is known in real time.

For example, the patent EP09425116 by the same applicant shows a computer-assisted system for guiding a surgical/diagnostic instrument in the body of a patient comprising a first marker device configured to be arranged integral with a region of the body of a patient and including first and second patient markers having a given mutual arrangement; a second marker configured to be coupled to the surgical/diagnostic instrument and including third instrument marker elements; an optical location sensor configured to locate the second and third marker elements in a first reference system; and a processing unit configured to acquire at least one tomography image of the region of the patient's body comprising the first marker elements in un second reference system different from the first, acquire the position of the second and third marker elements in the first reference system, and determine the position of said third marker elements in the second reference system based on a correlation between the first and the second reference systems.

The optical sensor generally comprises a pair of video cameras adapted to acquire, from different angles, an image of an area of vision in which the working/diagnostic instrument is located.

US2004147839 describes a method of registering an article having a surface to previously created scan data of the article, the method comprising the steps of: providing a flexible substrate having multiple tracking points attached to the substrate; applying the substrate to the article to be registered; creating a model of the surface of the article from a location of each tracking point; and registering the model with the previously created scan data.

US5999840 describes an image data registration system comprising: an image data storage unit for storing a first data set of three-dimensional image data associated with a predetermined portion of an object with reference to a first coordinate frame; a first image data acquisition and storage device for obtaining and storing a second data set of three-dimensional image data associated with a surface of said predetermined portion of said object with reference to a second coordinate frame; and a second image data acquisition and storage device for obtaining and storing a third data set of three-dimensional image data associated with a movable probe positioned in close proximity to said predetermined portion of said object with reference to a third coordinate frame; an image data processor for registering said first, second and third data sets of three-dimensional image data to generate a matched image data set of three-dimensional image data in which said first, second and third coordinate frames are relatively aligned with one another.

### SUBJECT MATTER AND SUMMARY OF THE INVENTION

The object of the present invention is to improve the system described in the aforesaid patent by producing an optical sensor that can perform further functions, such as scanning functions for the detection of three-dimensional profiles.

The preceding object is achieved by the present invention as it relates to a computer-assisted system for guiding a surgical/diagnostic instrument in the body of a patient arranged on a support structure as claimed in claim 1.

### BRIEF DESCRIPTION OF THE DRAWINGS

- Fig. 1 schematically shows a computer-assisted system for guiding a surgical instrument in the body of a patient according to the present invention;
- Fig. 2 shows a first detail of the system of Fig. 1;
- Figs. 3 and 4 show a second detail of the system of Fig. 1.

### DESCRIPTION

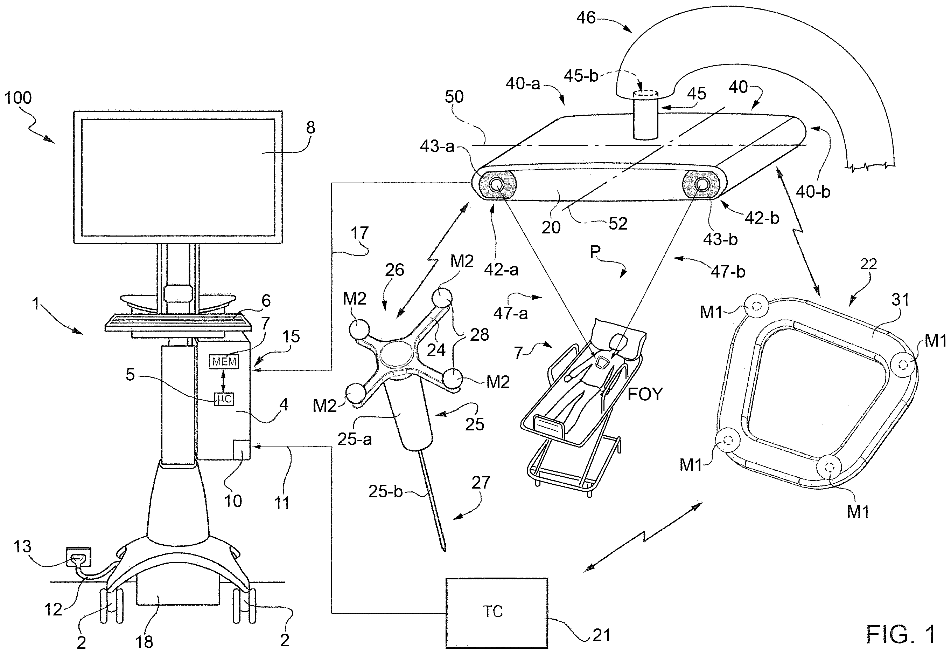

Fig. 1 shows a system according to the present invention comprising an instrumental station 1; an optical tracking sensor 20 produced according to the present invention and detailed below; in particular, an infrared sensor configured to cooperate with the instrumental station 1; a patient marker 22, provided with infrared marker elements M1 and configured to be arranged on a portion of the body of a patient P and to cooperate with the tracking sensor 20 (as better described hereunder); and an instrument marker 26, provided with a plurality of second infrared marker elements M2 (in the example spheres M2 opaque to infrared radiation that lie on the same plane and that have a predetermined arrangement relative to one another) and configured to be coupled to a surgical instrument 27 to cooperate with the infrared tracking sensor 20 (as better described below). The patient is arranged on a supporting structure 7 (typically a stretcher) that prevents shifting of the patient in space. The patient is normally anaesthetised or heavily sedated.

In particular, the surgical/diagnostic instrument 27 (in the example of Fig. 1 an endoscopic needle) comprises a grippable proximal portion 25a and an operative distal portion 25b; the instrument marker 26 is coupled to an intermediate portion, leaving the distal portion 25b free. It should be noted that, for greater clarity of representation, the relative sizes of the elements shown in the figures are not proportional to one another.

The instrumental station 1 (Fig. 1) comprises a processing unit 4, for example a processor of known type provided with a microcontroller 5 and with a memory 7, connected to each other; a data input user interface 6, for example including a keypad and mouse; a viewing interface 8, for example a high resolution LCD monitor; a network interface 10, configured to support a private and/or public network connection 11 (for example an Ethernet network).

The instrumental station 1 also comprises a power connection 12, configured to supply the instrumental station 1 with electrical power by means of a wall power socket 13; a tracking input 15, configured to support a connection 17 (either of wireless or wired type) between the instrumental station 1 and the optical tracking sensor 20; and an energy storage unit 18, for example a battery, connected to the wall socket 13 through the power connection 12 and configured to temporarily supply the instrumental station 1 in the event of an outage in the power supplied by the wall socket 13.

According to the embodiment shown, the processing unit 4 is a personal computer (PC), comprising an external protective casing housed on a shelf of the instrumental station 1 and integral with the instrumental station 1 during any movement of this latter on the wheels 2.

In order to ensure that the coordinates belonging to the surgical/diagnostic instrument 27 and acquired by the infrared/electromagnetic tracking system 20 always refer the body of the patient P, the patient marker 22 is arranged in contact with the patient so as to be integral therewith in a region of the patient (externally) close to the operating region.

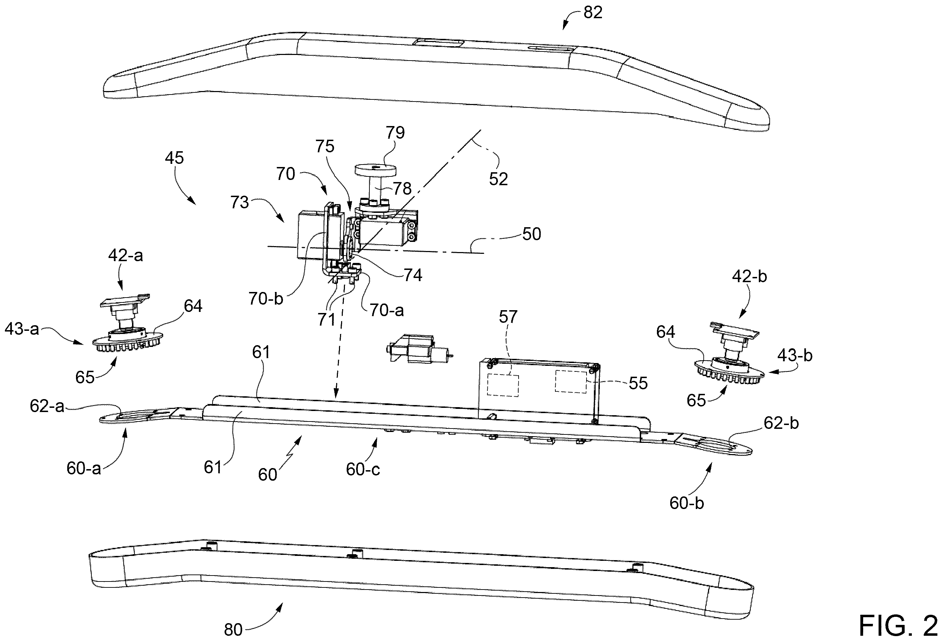

According to the embodiment shown, the patient marker 22 in the infrared version of Figs. 1 and 2, comprises a body 31, for example made of plastic material or, more in general, a material transparent to the image acquisition system used. In the example represented, the body 31 is in the shape of a trapezoidal frame with the spherical marker elements M1 arranged at the vertex of the trapezoid. Nonetheless, the shape of the marker element can differ, for example it can be Y-shaped or X-shaped, with the marker elements arranged at the ends of the arms.

The system operates as described in the patent EP09425116 by the same applicant; in particular, an initialising and calibration step is performed in which CT / MR images of an area of interest are acquired; these images are composited with one another using known algorithms in order to produce a three-dimensional image having its own reference system, the reference system of the three-dimensional image is aligned with that of the sensor 20 and, finally, an operating step is performed in which the processing unit 4 carries out a virtual navigation according to the modes described in EP09425116 using the aforesaid correctly positioned three-dimensional image as three-dimensional model.

In this way, the processing unit 4 allows the following to be viewed on the viewing unit 8:
- a three-dimensional representation of the region of the body generated based on the reconstructed and correctly positioned three-dimensional image; and
- a three-dimensional representation of the operational portion 25b of the surgical instrument 25, graphically superimposed on the three-dimensional representation of the region of the patient's body using the position of second marker elements and of the numerical model of the surgical instrument stored in the memory 7.

In this way, a system is provided that is user-friendly for the doctor oriented towards minimally invasive operations in the operating theatre.

During the phases of surgery, a three-dimensional model of the area of the patient being operated on, i.e. showing internal organs and tissues, is viewed on the viewing interface 8. As said, this three-dimensional model is generated by the processing unit 4.

In this way, it is possible to make the internal position of tissues according to the image obtained with a computerized tomography scan coherent with the current position during the operation.

The viewing interface 8 also shows a three-dimensional model of the surgical instrument used superimposed on the three-dimensional model of the area of the patient being operated on. The type of surgical instrument 25 to be viewed may be chosen by the doctor performing the operation from a plurality of possible models, previously created and stored in the memory 7 of the processing unit 4.

The doctor manipulating the surgical/diagnostic instrument 27 provided with the instrument marker 26 is guided by the images viewed on the viewing interface 8 throughout the entire operation. The trajectory of the surgical/diagnostic instrument 27 is calculated with the aid of artificial-intelligence algorithms so that the whole of the area involved is treated with the minimum number of insertions, ensuring total coverage without striking vital organs and/or obstacles. These organs are identified through the three-dimensional model shown on the viewing interface 8. The position of the surgical/diagnostic instrument 27 is entered in the three-dimensional model due to the measurement that the tracking sensor 20 performs on the spatial coordinates of the instrument marker 26.

The optical tracking sensor 20 comprises:
- a stereoscopic viewing system in which a first infrared ray video camera 42-a associated with a first infrared ray illuminator 43-a is carried by a first end 40-a of an elongated support element 40 and a second infrared ray video camera 42-b associated with a second infrared ray illuminator 43-b is carried by a second end 40-b of the elongated support element 40;
- a motorized pivot device 45 having a first end 45-a integral with the elongated support element 40 and a second end 45-b that can be fastened to a support structure 46 (partially shown) adapted to maintain the sensor 20 in a high position above the stretcher 7 on which the patient P is lying.

The video cameras 42-a and 42-b have respective axes 47-a and 47-b tilted towards each other and converging in an area of view FOV in which the patient P is located.

The motorized pivot device 45 has at least two degrees of freedom and produces the rotation of the support element 40 relative to the support structure 46 about a first axis (horizontal in the example) 50 (tilt) and according to a second axis 52 (pan) perpendicular to the first.

The instantaneous value of an angle **alpha** (relative to a vertical reference) about the tilt axis 50 and the instantaneous value of an angle **beta** (relative to a vertical reference) about the pan axis is detected by a solid state inertial sensor (inclinometer) 55 (of known type - Fig. 2) arranged on the support element 40. The signal produced by the inertial sensor 55 is sent to a microprocessor control unit 57 carried by the support element 40 and adapted to drive the actuators (shown below) that produce the rotations about the tilt axis 50 and the pan axis 52.

Control of the actuators that produce the movement of the pivot device about the tilt axis 50 and about the pan axis 52 enables the area of vision FOV to be moved in space; in particular - as will be explained in more detail below - the area of vision FOV is moved so as to maintain the image of the instrument marker 26 detected by the video camera 42-a/42-b substantially at the centre of the image detected also in the event of shifting of the instrument in order to perform a tracking function of the instrument marker 26 (and therefore of the surgical/diagnostic instrument 27) by the optical tracking sensor 20.

The implementation of movements about the tilt 50 and pan 52 axes enables the sensor 20 to track the operating tool so that it is always in the FOV, i.e. in the area in which the errors are lowest.

In greater detail (Fig. 2), the support element 40 comprises an elongated metal wall 60 having a pair of integral lips 61 that extend along the longer sides of a flat rectangular central portion 60-c of the elongated wall 60. The elongated wall 60 has end portions 60-a, 60-b of the elongated wall 60 of flat type integral with the central portion 60-c and arranged folded by a few degrees relative to the central portion 60-c. Each end portion 60-a, 60-b is provided with a circular through hole 62-a, 62-b in which there is housed a respective assembly comprising the first/second video camera 42-a, 42-b and the first/second illuminator 43-a,43-b. For example, the unit can comprise a flat wall 64 in the shape of a circular crown supporting, on a first face thereof, the LEDs 65 of the illuminator and provided with a video camera mounted coaxially to the circular crown shaped flat structure 64 (the axis of the video camera is coaxial to the circular crown).

The first end of the pivot element 45 is stably connected with the central portion 60-c.

The pivot device 45 comprises a first L-shaped bracket 70 provided with a first flat portion with a shorter side 70-a fastened by means of screws 71 on the central portion 60-b and a second flat portion with a longer side 70-b that carries a first actuator 73 of electrical type provided with a first rotating output member 74 that rotates about the first tilt axis 50.

An end portion of the first rotating output member 74 is fixed to a first flat portion of a second L-shaped bracket 75 parallel to the second flat portion 70-b. The second L-shaped bracket 75 comprises a second flat portion perpendicular to the first and fixed to an end portion of a second rotating output member (not visible in the figures) of a second electrical actuator 77. The second rotating output member extends from a casing of the second actuator 77 and rotates about the pan axis 52. The casing of the second actuator 77 is integral with a tubular sleeve 78, the circular end flange 79 of which produces the second end of the pivot 45. The pivot device 45 produced according to the present invention comprises a limited number of parts and for this reason is simple to produce, is rustic and sturdy and has limited costs. Moreover, the sizes of the pivot device 45 are limited.

The sensor 20 comprises an external protective casing that houses the metal wall 60 and the elements carried thereby. The protective casing comprises a lower shell 80 adapted to be fixed to a lower face of the wall 60 and provided with end holes (not shown) for the video cameras 42-a, 42-b and an upper half-shell 82 adapted to be fixed to an upper face of the wall 60 and provided with a central hole 84 engaged, in use, by the sleeve 78.

Preferably, the image processing operations for driving the actuators 73 and 77 are as follows:
a) two-dimensional coordinates (xa1, ya1 - xa2,ya2 - xa3,ya3) of the centre of the markers M2 (spheres) are determined on the images detected by the video cameras 42-a, 42-b as the single sphere that produces the marker M is viewed as a point in space;
b) the two images are composited to determine the three-dimensional coordinates (in the example a triplet xa1, ya1, za1 - xa2, ya2, za2 - xa3,ya3,za3 although this number can be different, for example four xa1, ya1, za1 - xa2, ya2, za2 - xa3,ya3,za3 - xa4,ya4,za4) of the centre of the markers M2 in space;
c) on the basis of the three-dimensional coordinates (xa1, ya1, za1 - xa2,ya2,za2 - xa3,ya3,za3) defined, the centroid of the markers and the three angles that together with the three coordinates identify the 6 degrees of freedom (DOF) that characterise the position of a rigid body (instrument marker 26) in space are calculated;
d) the operations a), b) and c) are repeated iteratively at successive instants so as to define the instantaneous value of a vector V that provides the position of the centroid and the direction and sense of its shift in space;
e) the position of the vector is compared with a three-dimensional threshold that defines a cuboid within which the vector must be contained to enable a good view of the surgical/diagnostic instrument in the FOV; if the three-dimensional threshold is not respected, the actuators are controlled to produce a shift of the centroid along the same direction and with a sense opposite to the one defined by the vector. The extent of the shift is such as to return the centroid within the three-dimensional threshold and enables good view of the surgical/diagnostic instrument in the FOV.

The tracking sensor is provided with:
- a first generator 90 adapted to emit a laser beam LS along a first direction transverse to the elongated support element 40 and that intersects the area of view FOV. The laser beam LS has a wavelength visible to the human eye and is provided to produce a laser spot (for example in the shape of a cross) visible on the body of the patient P that can be used in a procedure for adjustment of the sensor 20;
- a second generator 92 adapted to emit an infrared beam IR along a second direction transverse to the elongated support element 40 and that intersects the area of view FOV affecting the patient P.

There is also provided a deflection device that produces the movement of the infrared beam IR relative to the support structure 7 (the stretcher 7 in the example) so as to produce an IR spot (visible by the video cameras) that hits an area of the patient and moves in space performing the scan of this area. The intensity of the beam that produces the IR spot that hits the skin of the area of the patient is much greater than the intensity of the scattered infrared radiation generated by the first/second illuminator 43-a,43-b. In this way, the first/second video camera 42-a, 42-b generates images on which the image of the IR spot projected on the patient is fully distinguishable relative to the images of the markers M1 and M2.

Preferably, the deflection device can also produce the movement of the laser beam to move the laser spot on the patient's body.

The video cameras 42-a, 42-b are adapted to detect the image of the IR spot to detect (by means of algorithms of known type and therefore not further detailed) the distance between sensor 20 and spot and the position in space (x,y,z) of this spot. The control unit 57 is adapted to correlate the distance values measured for successive adjacent positions of the IR spot and repeat the scans for sections contiguous with one another (typically parallel) in order to obtain a three-dimensional profile of the area of interest.

According to the present invention, the infrared ray generator is integral with the support element 40 and emits an IR laser beam having a fixed predetermined tilt relative to the support element 40. In this case, the deflection device is produced by the pivot device 45 that performs the rotation of the support element 40 about the tilt 50 and pan 52 axes to adjust the position in space of the IR beam and move the IR spot to the area of the patient in order to perform a scan. The video cameras 42-a, 42-b detect the image of the IR spot and determine the distance between the sensor 20 and the position in space (x,y,z) of this spot. On the basis of these data, a three-dimensional profile of the area involved is obtained (by means of known algorithms) and stored.

In this way, the pan and tilt movements are exploited to obtain the profile of the area of interest of the patient. These profiles can be compared, by means of known comparison algorithms, with stored profiles obtained by previously detected CT/MR images. This avoids the use of manual trackers or trackers that require to be aligned with the coordinate system of the sensor 20. In fact, the reference between tracking sensor and viewing sensor is direct, as both functions are performed by the same device (the sensor 20).

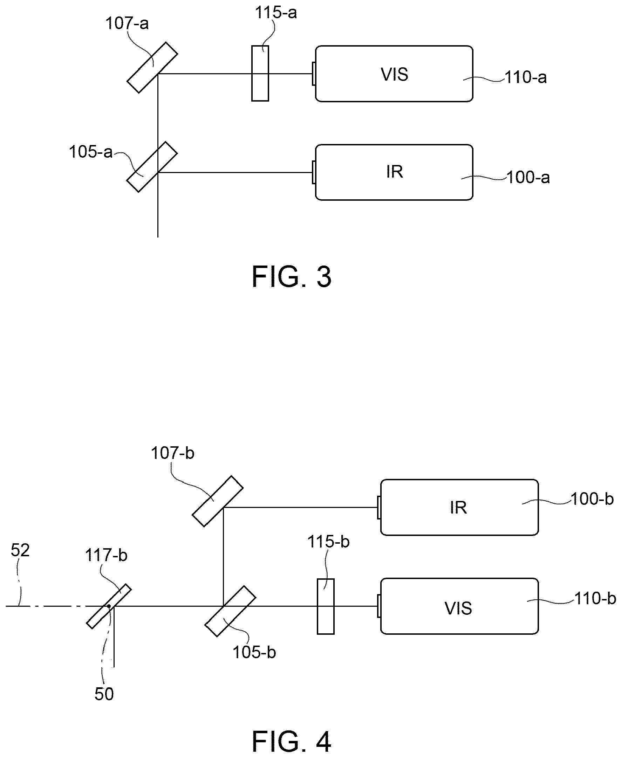

Fig. 3 shows in detail the optical system of the invention, which comprises a plurality of components carried by the support structure 46, in particular:
- a source 100-a configured to emit a beam of infrared rays that is fed to a first input of a beam splitter
- a laser source 110-b that emits a beam of visible light that is fed to a second input of the beam splitter 105-a after having passed through a pattern generator 115-a (for example the pattern produced can be a cross as indicated above) and has been reflected by 90° by a mirror 107-a.

The beam splitter 105-a is configured to output the beam of infrared rays and the laser beam having directions of propagation parallel to each other.

These beams move in space thanks to the movement of the pivot device 45.

According to an alternative example not covered by the attached claims and shown in Fig. 4, the infrared ray generator 90 is integral with the support element 40 and emits an IR laser beam having an adjustable tilt relative to the support element 40 by means of an optical deflection system. In this case, the pivot device 45 is not used to perform the scan as the optical deflection system adjusts the position in space of the IR beam and moves the IR spot to the area of the patient performing a scan. The video cameras 42-a, 42-b detect the image of the IR spot and determine the distance between the sensor 20 and the position in space (x,y,z) of this spot. On the basis of these data, a three-dimensional profile of the area involved is obtained and stored.

With particular reference to Fig. 4, the infrared ray generator 90 produced according to this alternative example comprises:
- a source 100-b configured to emit a beam of infrared rays that is fed to a first input of a beam splitter 105-b after being reflected by a mirror 107-b for infrared rays arranged tilted by 45° relative to the direction of propagation of the ray;
- a laser source 110-b that emits a beam of visible light that is fed to a second input of the beam splitter 105-b after having passed through a pattern generator 115-b (for example the pattern produced can be a cross, as indicated above); the beam splitter 105-b is configured to output the beam of infrared rays and the laser beam having directions of propagation parallel to each other;
- an oscillating mirror 117-b on which the infrared beam and the laser beam coming from the output of the beam splitter are incident. The oscillating mirror 117-b is configured to swivel about the horizontal tilt axis 50 and about the pan axis 52.
- Typically the oscillating mirror 117-b is produced using MEMS technology.

## Claims

1. Computer-assisted system for guiding a surgical/diagnostic instrument (27) in the body of a patient arranged on a support structure comprising:
- a first patient marker device (22) configured to be arranged in contact with the patient so as to be integral with a body region of a patient (P) to be treated by means of said surgical/diagnostic instrument (27) and including first marker elements (M1);
- a second instrument marker device (26) configured to be coupled to said surgical/diagnostic instrument (27) and including second marker elements (M2);
- an optical tracking sensor (20) configured to locate said first and second marker elements (M1, M2); and
- a processing unit (4) connected to the optical tracking sensor (20) and adapted to perform a virtual navigation based on an imported/reconstructed three-dimensional image and on the image detected by the optical tracking sensor (20) for viewing on a viewing unit (8):
- a three-dimensional representation of the body region generated based on said three-dimensional image repositioned relative to the optical tracking sensor (20); and
- a three-dimensional representation of at least one operating portion (25b) of the surgical/diagnostic instrument (27), graphically superimposed on the three-dimensional representation of the body region of the patient using the position of the second marker elements (M2) and of a model of the surgical instrument,
said optical tracking sensor (20) being provided with a stereoscopic viewing system in which a first infrared ray video camera (42-a) associated with a first infrared ray illuminator (43-a) is carried by a first end (40-a) of an elongated support element (40) and a second infrared ray video camera (42-b) associated with a second infrared ray illuminator (43-b) is carried by a second end (40-b) of the elongated support element (40); the video cameras (42-a and 42-b) having respective axes (47-a and 47-b) tilted towards each other and converging in an area of view (FOV) of the optical tracking sensor (20); said optical tracking sensor comprising a microprocessor control unit (57); said optical tracking sensor (20) being provided with a generator adapted to emit an infrared beam along a direction that is transverse to the elongated support element (40) and intersects the area of view (FOV) affecting the patient (P); the system also comprising a deflection device that produces the movement of the infrared beam relative to the elongated support element (40) so as to produce an infrared spot that hits an area of the patient and moves in space performing the scan of this area; said video cameras (42-a, 42-b) being adapted to detect the position in space (x,y,z) of this spot and the microprocessor control unit (57) being adapted to detect the distance between optical tracking sensor (20) and position in space (x,y,z) of the spot; the microprocessor control unit (57) being adapted to correlate the distance values measured for successive adjacent positions of the spot and repeat the scans for sections contiguous with one another in order to obtain a three-dimensional profile of the area of interest,
**characterised in that** the optical tracking sensor (20) also comprises a motorized pivot device (45) having a first end (45-a) integral with the elongated support element (40) and a second end (45-b) that can be fastened to a support structure (46); said motorized pivot device has at least two degrees of freedom and produces the rotation of the elongated support element (40) relative to the support structure (46) about a first horizontal tilt axis (50) and according to a second horizontal pan axis (52) perpendicular to the first horizontal tilt axis (50); the microprocessor control unit (57) is configured to drive the actuators that produce the rotations about the first horizontal tilt axis (50) and the second horizontal pan axis (52) to move the area of view in space; the infrared ray generator is integral with the elongated support element (40) and emits an infrared laser beam having a predetermined tilt relative to the elongated support element (40); the deflection device consists of the motorized pivot device (45) that performs the rotation of the elongated support element (40) about the first horizontal tilt (50) and the second horizontal pan (52) axes to adjust the position in space of the infrared beam and move the infrared spot to the area of the patient in order to perform a scan.

2. System according to claim 1, wherein said generator comprises an optical system carried by the support element and comprising:
- a source (100-a) configured to emit a beam of infrared rays that is fed to a first input of a beam splitter (105-a);
- a laser source (110-a) that emits a beam of visible light that is fed to a second input of the beam splitter (105-a) after having passed through a pattern generator (115-a); the beam splitter (105-a) is configured to output the beam of infrared rays and the laser beam having directions of propagation parallel to each other.

3. System according to any one of the preceding claims wherein the microprocessor control unit (57) is configured to move the area of view and maintain the image of the instrument marker (26) detected by the video camera (42-a/42-b) substantially at the centre of the same image also in the case of shifting of the instrument in order to perform a tracking function of the instrument marker by the optical tracking sensor (20).

4. System according to claim 1, wherein there is provided an inertial sensor (55), in particular an inclinometer, carried by the elongated support element (40) and adapted to detect the instantaneous value of an angle **alpha** about the first horizontale tilt axis (50) relative to a reference and the instantaneous value of an angle **beta** about the second horizontal pan axis (52) relative to a reference; the signal produced by the inertial sensor (55) is sent to said microprocessor control unit (57).

5. System according to any one of the dependent claims, wherein the microprocessor control unit (57) is carried by the elongated support element (40).

6. System according to any one of the preceding claims, wherein the elongated support element (40) comprises an elongated wall (60) comprising a flat rectangular central portion (60-c) and flat end portions (60-a, 60-b) arranged tilted relative to the central portion (60-c); each end portion (60-a, 60-b) is provided with a through hole (62-a, 62-b) in which there is housed a respective assembly comprising the first/second video camera (42-a, 42-b) and the first/second illuminator (43-a,43-b); the first end of the motorized pivot device (45) is stably connected with the central portion (60-c).

7. System according to claim 6, wherein each assembly comprises a flat support wall (64) in the shape of a circular crown supporting, on a first face thereof, a plurality of LEDs (65) of the illuminator and provided with a video camera mounted coaxially to the circular crown shaped flat support wall (64).

8. System according to claim 1, wherein the motorized pivot device (45) comprises a first L-shaped bracket (70) provided with a first flat portion with a shorter side (70-a) fastened on the central portion (60-b) and a second flat portion with a longer side (70-b) that carries a first actuator (73) of electrical type provided with a first rotating output member (74) that rotates about the first horizontal tilt axis (50);
an end portion of the first rotating output member (74) is fixed to a first flat portion of a second bracket (75) that comprises a second flat portion perpendicular to the first portion and fixed to an end portion of a second rotating output member of a second electrical actuator (77); the second rotating output member extends from a casing of the second electrical actuator (77) and rotates about the second horizontale pan axis (52);
the casing of the second electrical actuator (77) is integral with an element that produces the second end of the motorized pivot device (45).

9. System according to any one of the preceding claims, wherein said optical tracking sensor (20) is provided with a first generator adapted to emit a laser beam LS along a first direction that is transverse to the elongated support element (40) and intersects the area of view (FOV); the laser beam LS has a wavelength visible to the human eye and is provided to produce a laser spot visible on the body of the patient P that can be used in a procedure for adjustment of the optical tracking sensor (20).

## Patentansprüche

1. Computergestütztes System zum Führen eines chirurgischen/diagnostischen Instruments (27) im Körper eines auf einer Trägerstruktur angeordneten Patienten, aufweisend:
- eine erste Patientenmarkierung-Vorrichtung (22), die konfiguriert ist, in Kontakt mit dem Patienten angeordnet zu werden, um integral mit einer mit Hilfe des chirurgischen/diagnostischen Instruments (27) zu behandelnden Körperregion eines Patienten (P) zu sein, und erste Markierungselemente (M1) aufweist;
- eine zweite Instrumentenmarkierung-Vorrichtung (26), die konfiguriert ist, mit dem chirurgischen/diagnostischen Instrument (27) gekoppelt zu werden und zweite Markierungselemente (M2) aufweist;
- einen optischen Verfolgungssensor (20), der konfiguriert ist, die ersten und zweiten Markierungselemente (M1, M2) zu lokalisieren; und
- eine Verarbeitungseinheit (4), die mit dem optischen Verfolgungssensor (20) gekoppelt ist und angepasst ist, auf Basis eines importierten/rekonstruierten dreidimensionalen Bilds und auf Basis des von dem optischen Verfolgungssensor (20) detektierten Bilds eine virtuelle Navigation durchzuführen, um auf einer Betrachtungseinheit (8) zu betrachten:
- eine dreidimensionale Darstellung der Körperregion, erzeugt auf Basis des relativ zu dem optischen Verfolgungssensor (20) neu positionierten dreidimensionalen Bilds; und
- eine dreidimensionale Darstellung mindestens eines Operationsteils (25b) des chirurgischen/diagnostischen Instruments (27), graphisch überlagert auf die dreidimensionale Darstellung der Körperregion des Patienten unter Verwendung der Position der zweiten Markierungselemente (M2) und eines Modells des chirurgischen Instruments,
wobei der optische Verfolgungssensor (20) mit einem stereoskopischen Betrachtungssystem ausgestattet ist, in welchem eine erste Infrarotstrahlung-Videokamera (42-a), die mit einem ersten Infrarotstrahlung-Illuminator (43-a) kombiniert ist, von einem ersten Ende (40-a) eines länglichen Trägerelements (40) getragen wird, und eine zweite Infrarotstrahlung-Videokamera (42-b), die mit einem zweiten Infrarotstrahlung-Illuminator (43-b) kombiniert ist, von einem zweiten Ende (40-b) des länglichen Trägerelements (40) getragen wird;
wobei die Videokameras (42-a und 42-b) jeweilige Achsen (47-a und 47-b) haben, die zueinander geneigt sind und in einem Betrachtungsfeld (FOV) des optischen Verfolgungssensors (20) konvergieren;
wobei der optische Verfolgungssensor eine Mikroprozessor-Steuereinheit (57) aufweist; wobei der optische Verfolgungssensor (20) mit einem Generator versehen ist, der angepasst ist, einen Infrarotstrahl entlang einer Richtung zu emittieren, die transversal zu dem länglichen Trägerelement (40) ist und das Betrachtungsfeld (FOV) kreuzt, den Patienten (P) treffend;
wobei das System außerdem eine Ablenkungsvorrichtung aufweist, die die Bewegung des Infrarotstrahls relativ zu dem länglichen Trägerelement (40) erzeugt, um so einen Infrarotfleck zu erzeugen, der auf ein Gebiet des Patienten trifft und sich im Raum bewegt, um eine Abtastung dieses Gebiets durchzuführen;
wobei die Videokameras (42-a, 42-b) angepasst sind, die räumliche Position (x, y, z) dieses Flecks zu detektieren, und die Mikroprozessor-Steuereinheit (57) angepasst ist, den Abstand zwischen dem optischen Verfolgungssensor (20) und der räumlichen Position (x, y, z) des Flecks zu detektieren;
wobei die Mikroprozessor-Steuereinheit (57) angepasst ist, die gemessenen Abstandswerte aufeinanderfolgender benachbarter Positionen des Flecks zu korrelieren und die Abtastungen für aneinander angrenzende Abschnitte zu wiederholen, um ein dreidimensionales Profil des Gebiets von Interesse zu gewinnen,
**dadurch gekennzeichnet, dass**
der optische Verfolgungssensor (20) außerdem eine motorisierte Drehvorrichtung (45) aufweist, die ein mit dem länglichen Trägerelement (40) integrales erstes Ende (45-a) und ein zweites Ende (45-b) hat, das an einer Trägerstruktur (46) befestigt werden kann;
die motorisierte Drehvorrichtung mindestens zwei Freiheitsgrade hat und die Drehung des länglichen Trägerelements (40) relativ zu der Trägerstruktur (46) um eine erste horizontale Kippachse (50) und gemäß einer zweiten horizontalen Schwenkachse (52), die senkrecht zu der ersten horizontalen Kippachse (50) ist, bewirkt;
die Mikroprozessor-Steuereinheit (57) konfiguriert ist, die Aktuatoren anzutreiben, die die Drehungen um die erste horizontale Kippachse (50) und die zweite horizontale Schwenkachse (52) bewirken, um das Betrachtungsfeld im Raum zu bewegen;
der Infrarotstrahlungsgenerator integral mit dem länglichen Trägerelement (40) ist und einen infraroten Laserstrahl emittiert, der eine vorgegebene Neigung relativ zu dem länglichen Trägerelement (40) hat;
die Ablenkungsvorrichtung aus der motorisierten Drehvorrichtung (45) besteht, die die Drehung des länglichen Trägerelements (40) um die erste horizontale Kippachse (50) und die zweite horizontale Schwenkachse (52) durchführt, um die räumliche Position des Infrarotstrahls anzupassen und den Infrarotfleck in das Gebiet des Patienten zu bewegen, um eine Abtastung durchzuführen.

2. System nach Anspruch 1, wobei
der Generator ein optisches System aufweist, das von dem Trägerelement getragen wird und aufweist:
- eine Quelle (100-a), die konfiguriert ist, einen Infrarotstrahl zu emittieren, der einem ersten Eingang eines Strahlteilers (105-a) zugeführt wird;
- eine Laserquelle (110-a), die einen Strahl sichtbaren Lichts emittiert, der nach Durchgang durch einen Mustergenerator (115-a) einem zweiten Eingang des Strahlteilers (105-a) zugeführt wird; und
der Strahlteiler (105-a) konfiguriert ist, den Infrarotstrahl und den Laserstrahl derart auszugeben, dass sie zueinander parallele Ausbreitungsrichtungen haben.

3. System nach einem der vorstehenden Ansprüche, wobei die Mikroprozessor-Steuereinheit (57) konfiguriert ist, das Betrachtungsfeld zu bewegen und das von der Videokamera (42-a, 42-b) detektierte Bild der Instrumentenmarkierung (26), auch im Fall eines Verschiebens des Instruments, im Wesentlichen in der Mitte des gleichen Bilds zu halten, um mit Hilfe des optischen Verfolgungssensors (20) die Instrumentenmarkierung zu verfolgen.

4. System nach Anspruch 1, wobei ein Trägheitssensor (55), insbesondere ein Inklinationsmesser, bereitgestellt ist, der von dem länglichen Trägerelement (40) getragen wird und angepasst ist, den momentanen Wert eines Winkels alpha um die erste horizontale Kippachse (50) relativ zu einer Referenz und den momentanen Wert eines Winkels beta um die zweite horizontale Schwenkachse (52) relativ zu einer Referenz zu detektieren; wobei das von dem Trägheitssensor (55) erzeugte Signal zu der Mikroprozessor-Steuereinheit (57) geschickt wird.

5. System nach einem der abhängigen Ansprüche, wobei die Mikroprozessor-Steuereinheit (57) von dem länglichen Trägerelement (40) getragen wird.

6. System nach einem der vorstehenden Ansprüche, wobei
das längliche Trägerelement (40) eine längliche Wand (60) aufweist, die einen flachen rechteckigen zentralen Abschnitt (60-c) und flache Endabschnitte (60-a, 60-b), die relativ zu dem zentralen Abschnitt (60-c) geneigt sind, aufweist;
jeder Endabschnitt (60-a, 60-b) mit einem Durchgangsloch (62-a, 62-b) versehen ist, in welchen eine jeweilige Anordnung untergebracht ist, die die erste/zweite Videokamera (42-a, 42-b) und den ersten/zweiten Illuminator (43-a, 43-b) aufweist; und
das erste Ende der motorisierten Drehvorrichtung (45) fest mit dem zentralen Abschnitt (60-c) verbunden ist.

7. System nach Anspruch 6, wobei jede Anordnung eine flache kranzförmige Trägerwand (64) aufweist, die an einer ersten Fläche mehrere LEDs (65) des Illuminators trägt und mit einer Videokamera versehen ist, die koaxial an der kranzförmigen flachen Trägerwand (64) angebracht ist.

8. System nach Anspruch 1, wobei
die motorisierte Drehvorrichtung (45) eine erste L-förmige Klammer (70) aufweist, die mit einem an dem zentralen Abschnitt (60-b) befestigten ersten flachen Abschnitt mit einer kürzeren Seite (70-a) und einem zweiten flachen Abschnitt mit einer längeren Seite (70-b) versehen ist, der einen ersten elektrischen Aktuator (73) trägt, der mit einem ersten rotierenden Abtriebselement (74) versehen ist, das um die erste horizontale Kippachse (50) rotiert;
ein Endabschnitt des ersten rotierenden Abtriebselements (74) an einem ersten flachen Abschnitt einer zweiten Klammer (75) befestigt ist, die einen zweiten flachen Abschnitt aufweist, der senkrecht zu dem ersten Abschnitt ist und an einem Endabschnitt eines zweiten rotierenden Abtriebselements eines zweiten elektrischen Aktuators (77) befestigt ist;
das zweite rotierende Abtriebselement sich von einem Gehäuse des zweiten elektrischen Aktuators (77) aus erstreckt und um die zweite horizontale Schwenkachse (52) rotiert;
das Gehäuse des zweiten elektrischen Aktuators (77) integral mit einem Element ist, das das zweite Ende der motorisierten Drehvorrichtung (45) erzeugt (bildet).

9. System nach einem der vorstehenden Ansprüche, wobei
der optische Verfolgungssensor (20) mit einem ersten Generator ausgestattet ist, der angepasst ist, einen Laserstrahl LS entlang einer ersten Richtung zu emittieren, die transversal zu dem länglichen Trägerelement (40) ist und das Betrachtungsfeld (FOV) kreuzt;
der Laserstrahl LS eine für das menschliche Auge sichtbare Wellenlänge hat und bereitgestellt ist, um einen auf dem Körper des Patienten P sichtbaren Laserfleck zu erzeugen, der in einem Verfahren zur Justierung des optischen Verfolgungssensors (20) verwendet werden kann.

## Revendications

1. Système assisté par ordinateur pour guider un instrument chirurgical / de diagnostic (27) dans le corps d'un patient disposé sur une structure de support comprenant:
- un premier dispositif marqueur de patient (22) configuré pour être disposé en contact avec le patient de manière à être solidaire d'une région du corps d'un patient (P) à traiter au moyen dudit instrument chirurgical / de diagnostic (27) et comprenant des premiers éléments marqueurs (M1);
- un second dispositif marqueur d'instrument (26) configuré pour être couplé audit instrument chirurgical / de diagnostic (27) et comprenant des seconds éléments marqueurs (M2);
- un capteur optique de suivi (20) configuré pour localiser lesdits premier et second éléments marqueurs (M1, M2); et
- une unité de traitement (4) connectée au capteur optique de suivi (20) et adaptée pour effectuer une navigation virtuelle à partir d'une image tridimensionnelle importée / reconstruite et sur l'image détectée par le capteur optique de suivi (20) pour visualiser sur une unité de visualisation (8) :
- une représentation tridimensionnelle de la région du corps générée sur la base de ladite image tridimensionnelle repositionnée par rapport au capteur optique de suivi (20); et
- une représentation tridimensionnelle d'au moins une partie opérationnelle (25b) de l'instrument chirurgical / diagnostic (27), superposée graphiquement sur la représentation tridimensionnelle de la région du corps du patient en utilisant la position des seconds éléments marqueurs (M2) et d'un modèle de l'instrument chirurgical, ledit capteur optique de suivi (20) étant muni d'un système de visualisation stéréoscopique dans lequel une première caméra vidéo à rayons infrarouges (42-a) associée à un premier illuminateur de rayons infrarouges (43-a) est portée par une première extrémité (40-a) d'un élément de support allongé (40), et une seconde caméra vidéo à rayons infrarouges (42-b) associée à un second illuminateur de rayons infrarouges (43-b) est portée par une seconde extrémité (40 -b) de l'élément de support allongé (40); les caméras vidéo (42-a et 42-b) ayant des axes respectifs (47-a et 47-b) inclinés l'un vers l'autre et convergeant dans une zone de vision (FOV) du capteur optique de suivi (20); ledit capteur optique de suivi (20) comprenant une unité de commande à microprocesseur (57); ledit capteur optique de suivi (20) étant doté d'une générateur adapté pour émettre un rayon infrarouge IR le long d'une direction qui est transversale à l'élément de support allongé (40) et coupe la zone de vision (FOV) affectant le patient (P); le système comportant également un dispositif de déviation qui produit le mouvement du faisceau infrarouge par rapport à l'élément de support allongé (40) de manière à produire un point infrarouge qui frappe une zone du patient et se déplace dans l'espace en effectuant l'analyse de cette zone; les caméras vidéo (42-a, 42-b) étant adaptées pour détecter la position dans l'espace (x, y, z) de ce point ; l'unité de commande à microprocesseur (57) étant adaptée pour corréler les valeurs de distance mesurées pour les positions adjacentes successives du point et répéter les analyses pour des sections contiguës les unes aux autres afin d'obtenir un profil tridimensionnel de la zone d'intérêt,
**caractérisé en ce que** le capteur optique de suivi (20) comprend également un dispositif de pivotement motorisé (45) ayant une première extrémité (45-a) solidaire de l'élément de support allongé (40) et une seconde extrémité (45-b) qui peut être fixée à une structure de support (46); ledit dispositif de pivotement motorisé a au moins deux degrés de liberté et produit la rotation de l'élément de support allongé (40) par rapport à la structure de support (46) autour d'un premier axe d'inclinaison horizontal (50) et selon un deuxième axe horizontal (52) perpendiculaire au premier axe d'inclinaison horizontal (50); l'unité de commande à microprocesseur (57) est configurée pour entraîner les actionneurs qui produisent les rotations autour du premier axe d'inclinaison horizontal (50) et du second axe horizontal (52) pour déplacer la zone de vision dans l'espace; le générateur de rayons infrarouges est solidaire de l'élément de support allongé (40) et émet un faisceau laser infrarouge ayant une inclinaison prédéterminée par rapport à l'élément de support allongé (40); le dispositif de déviation est consiste en le dispositif de pivotement motorisé (45) qui effectue la rotation de l'élément de support allongé (40) autour des premier axe d'inclinaison horizontal (50) et second axe horizontal (52) pour régler la position dans l'espace du faisceau infrarouge et déplacer le point infrarouge dans la zone du patient afin d'effectuer une analyse.

2. Système selon la revendication 1, dans lequel le générateur comporte un système optique porté par l'élément de support et comprenant:
- une source (100-a) configurée pour émettre un faisceau de rayons infrarouges alimenté à une première entrée d'un diviseur de faisceau (105 -a);
- une source laser (110-a) qui émet un faisceau de lumière visible qui est alimenté à une deuxième entrée du diviseur de faisceau (105-a) après avoir traversé un générateur de motifs (115-a); le diviseur de faisceau (105-a) est configuré pour délivrer le faisceau de rayons infrarouges et le faisceau laser ayant des directions de propagation parallèles les unes aux autres.

3. Système selon l'une quelconque des revendications précédentes, dans lequel l'unité de commande à microprocesseur (57) est configurée pour déplacer la zone de vision et maintenir l'image du marqueur d'instrument (26) détectée par la caméra vidéo (42-a / 42-b) sensiblement au centre de la même image également dans le cas du décalage de l'instrument afin d'assurer une fonction de poursuite du marqueur d'instrument par le capteur optique de suivi (20).

4. Système selon la revendication 1, dans lequel il est prévu un capteur inertiel (55), en particulier un inclinomètre, porté par l'élément de support allongé (40) et adapté à détecter la valeur instantanée d'un angle alpha par rapport au premier axe d'inclinaison (50) relatif à une référence et la valeur instantanée d'un angle béta autour du second axe horizontal (52) par rapport à une référence; le signal produit par le capteur inertiel (55) est envoyé à ladite unité de commande à microprocesseur (57).

5. Système selon l'une quelconque des revendications dépendantes, dans lequel l'unité de commande à microprocesseur (57) est portée par l'élément de support allongé (40).

6. Système selon l'une quelconque des revendications précédentes, dans lequel l'élément de support allongé (40) comprend une paroi allongée (60) comprenant une partie centrale rectangulaire plate (60-c) et des parties d'extrémité plates (60-a, 60-b) disposées inclinées par rapport à la partie centrale (60-c); chaque partie d'extrémité (60-a, 60-b) est pourvue d'un trou traversant (62-a, 62-b) dans lequel est logé un ensemble respectif comprenant la première / seconde caméra vidéo (42-a, 42-b) et le premier / second illuminateur (43-a, 43-b); la première extrémité de l'élément de pivotement motorisé (45) est reliée de manière stable à la partie centrale (60-c).

7. Système selon la revendication 6, dans lequel chaque ensemble comporte une paroi de support plate (64) en forme de couronne circulaire, sur une première face de laquelle, est agencée une pluralité de DEL (65) de l'illuminateur, et une caméra vidéo est montée coaxialement avec le support plat en forme de couronne circulaire (64).

8. Système selon la revendication 1, dans lequel le dispositif de pivotement motorisé (45) comporte un premier support en forme de L (70) muni d'une première partie plate avec un côté plus court (70-a) fixé sur la partie centrale (60-b) et une deuxième partie plate avec un côté plus long (70-b) qui porte un premier actionneur (73) de type électrique pourvu d'un premier élément de sortie rotatif (74) qui tourne autour du premier axe d'inclinaison horizontal (50); une partie d'extrémité du premier élément de sortie en rotation (74) est fixée à une première partie plate d'un second support (75) qui comprend une seconde partie plate perpendiculaire à la première partie et est fixée à une partie d'extrémité d'un second élément de sortie rotatif d'un second actionneur électrique (77); le deuxième élément de sortie rotatif s'étend depuis un boîtier du second actionneur électrique (77) et tourne autour du second axe horizontal (52); le boîtier du second actionneur électrique (77) est solidaire d'un élément qui constitue la seconde extrémité du dispositif de pivotement motorisé (45).

9. Système selon l'une quelconque des revendications précédentes, dans lequel ledit capteur optique de suivi (20) est doté d'un premier générateur adapté pour émettre un faisceau laser LS selon une première direction qui est transversale à l'élément de support allongé (40) et coupe la zone de vision (FOV); le faisceau laser LS a une longueur d'onde visible à l'oeil humain et est prévu pour engendrer un point laser visible sur le corps du patient P qui peut être utilisé dans une procédure de réglage du capteur optique de suivi (20).
